(19) Europäisches Patentamt / European Patent Office / Office européen des brevets

(11) EP 4 527 191 A1

(12) **EUROPEAN PATENT APPLICATION**

(43) Date of publication:
**26.03.2025 Bulletin 2025/13**

(21) Application number: **23198667.0**

(22) Date of filing: **20.09.2023**

(51) International Patent Classification (IPC):
***A01K 1/015*** $^{(2006.01)}$ ***A61K 8/362*** $^{(2006.01)}$
***A61L 9/01*** $^{(2006.01)}$ ***A61L 15/20*** $^{(2006.01)}$
***A61Q 5/02*** $^{(2006.01)}$ ***A61Q 15/00*** $^{(2006.01)}$
***E03D 9/00*** $^{(2006.01)}$ ***A61K 8/41*** $^{(2006.01)}$
***A61K 8/46*** $^{(2006.01)}$ ***A61K 8/55*** $^{(2006.01)}$
***A61L 9/05*** $^{(2006.01)}$

(52) Cooperative Patent Classification (CPC):
**A61L 15/20; A01K 1/0155; A61K 8/44; A61K 8/55; A61L 9/01; A61L 15/46; A61Q 13/00;** A61L 9/05; A61L 2209/21

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Symrise AG**
**37603 Holzminden Niedersachsen (DE)**

(72) Inventors:
- **Dr. NIKOLAICZYK, Vanessa**
  **31848 Bad Münder am Deister (DE)**
- **WIESEL, Ece**
  **37603 Holzminden (DE)**
- **DIETRICH, Sandra**
  **37671 Höxter (DE)**
- **DR. DIAZ, Edison**
  **38642 Goslar (DE)**

(74) Representative: **Daniels, Stefanie Lisa**
**IP2 Patentanwalts GmbH**
**Schlossstraße 523-525**
**41238 Mönchengladbach (DE)**

(54) **UREASE INHIBITORS**

(57) Suggested is the use of a mixture consisting of
(a) at least one fragrance raw material and
(b) EDTA and/or NBPT
for inhibiting urease.

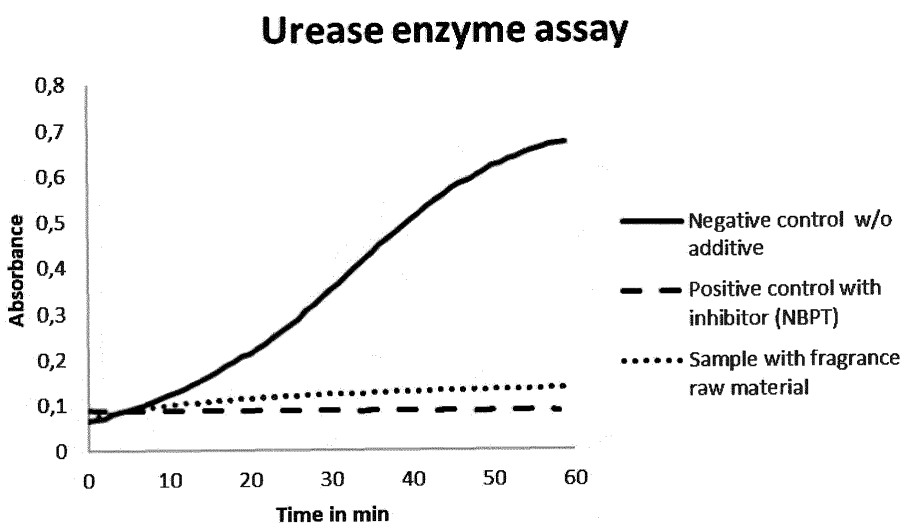


Figure (1/7)

EP 4 527 191 A1

## Description

### AREA OF INVENTION

The present invention relates to the field of functional perfumery, in particular to the prevention of ammonia malodor formation, more particular to a mixture which has the ability to inhibit the enzyme urease.

### BACKGROUND OF THE INVENTION

Fragranced products such as perfumes, deodorants or other fragrance-containing compositions have widely been used in personal care, home care or pet care products to mask unpleasant odors. Overcoming ammonia malodors however is a major challenge due to its ability to not only activate the olfactory system but also trigger trigeminal chemo sensation. The latter is known to be a warning system for exposure to chemical hazards (in this case due to changes of pH) and therefore the perception is very unpleasant and hard to mask by conventional fragrances. As a result, the best approach to counteract ammonia odors is to prevent their formation.

Ammonia can be formed by an enzymatic hydrolysis in which the enzyme urease converts the urea present in the urine of humans and animals into ammonia and carbamate. The latter reacts spontaneously to form another ammonia molecule and carbon dioxide. The enzyme urease can be of bacterial origin, as in the case of urine, but can also be produced by fungi, yeasts and plants. Since ammonia is formed over time by the enzymatic process, one can either use antibacterial agents to reduce the amount of urease-positive bacteria or chemically inhibit the enzyme urease to prevent the formation of ammonia.

Inhibition of urease has been thoroughly studied from a medicinal point of view but also for agricultural products in particular urea containing fertilizers. In both areas, very powerful small molecule inhibitors were developed that are highly specific and effective against urease. For example, known inhibitors include compounds containing urea fragments, flavonoids, quinolones, heterocyclic compounds, organophosphorus compounds, and coordination complexing agents.

However, many of these compounds cannot be used for personal care, home care and pet care compositions because they are either toxic in high concentrations or have inherent odors that affect the olfactory profile.

As a result, it was the object of the invention to provide improved urease inhibitors that can be readily used in consumer products to tackle the challenge of ammonia malodor formation.

### DESCRIPTION OF THE INVENTION

A first object of the present invention refers to the use of a mixture consisting of

(a) at least one fragrance raw material and

(b) EDTA and/or NBPT

for inhibiting urease.

The inventors have surprisingly found that the combination of fragrance raw materials together with the complexing agent Ethylenediaminetetraacetic acid (EDTA) and/or N-(n-Butyl)thiophosphoric triamide (NBPT) leads to a synergistic effect on urease inhibition. This is very surprising since EDTA or NBPT themselves have been previously described as urease inhibitors. The simultaneous use of different urease inhibitors is usually problematic, as they often hinder each other's effectiveness and this often leads to a deterioration in the urease inhibition instead of an improvement. However, the inventors have found that the combination of fragrance raw materials with EDTA, or the combination of fragrance raw materials with NBPT, or the combination of fragrance raw materials with EDTA and NBPT not only leads to an improvement of urease inhibition compared to the individual substances, but to a synergistic improvement.

Thus, the present invention enables the reduction or complete elimination of unpleasant smelling and/or possibly toxic other urease inhibitors in compositions, in particular in home care, personal care and pet care compositions. The inventors additionally found that the fragrance raw materials are also able to scavenge the ammonia which was already formed by chemical reaction. Therefore, the present invention is preferably also directed to a combination of initial inhibition of the malodor source with interception of the formed malodor by chemical reaction, which is an effective technology reduce or eliminate ammonia perception. In other words, preferably the present invention is also directed to the use of the mixture for inhibiting urease and scavenging ammonia which has already been formed.

Ureases functionally belong to the superfamily of amidohydrolases and phosphotriesterases. Ureases are found in numerous bacteria, fungi, algae, plants, and some invertebrates, as well as in soils, as a soil enzyme. They are nickel-containing metalloenzymes of high molecular weight. These enzymes catalyze the hydrolysis of urea into carbon dioxide and ammonia:

$$(NH_2)_2CO + H_2O \xrightarrow{urease} CO_2 + 2NH_3$$

The hydrolysis of urea occurs in two stages. In the first stage, ammonia and carbamate are produced. The carbamate spontaneously and rapidly hydrolyzes to ammonia and carbonic acid. Urease activity increases the pH of its environment as ammonia is produced, which is basic.

The fragrance raw materials as a part of the mixture according to the present invention are advantageously easily accessible or are readily available or can be easy prepared, are highly effective even at low concentrations commonly used for the application, in particular at concentrations at which the fragrance raw materials have no odor or at least only a barely perceptible odor, are largely or completely colorless, have a high stability in various mixtures or preparations and have no toxic and/or allergenic effect on humans.

The fragrance raw materials of the present invention also have the advantage that they can be combined with different fragrance raw materials, other than the fragrance raw materials according to the invention, and perfume oils or essential oils or usual components of a perfume oil in order to perfume products with any desired fragrance. Consequently, by the present invention, a wide range of fragrance types can be offered to consumers. In one embodiment according to the invention, only the fragrance raw materials of the present invention are used together with EDTA and/or NBPT to perfume products.

A "urease inhibitor" or "urease inhibitors" according to the present invention are chemical compounds, in the sense of the present invention in particular fragrance raw materials and EDTA and/or NBPT, that reduce or completely prevent the activity of the enzyme urease.

The fragrance raw materials as well as EDTA and NBPT can be in liquid form, undiluted or diluted with a solvent and are advantageously used for perfuming. Preferred solvents for this purpose are ethanol, isopropanol, diethylene glycol monoethyl ether, glycerol, propylene glycol, 1 ,2-butylene glycol, dipropylene glycol, diethyl phthalate, triethyl citrate, isopropyl myristate, triacetin, 2,2-dimethyl-1,3-dioxolane-4-methanol and diacetin.

In a preferred embodiment according to the invention, the amount of the mixture according to the invention is not sufficient to impart an inherent odor when added to a composition or product. The mixture according to the invention has the advantage that, when used, in particular according to the use according to the invention, it is already active for the purposes of the present invention in such low concentrations at which no or at least no perceptible intrinsic odor of the mixture is imparted. This allows the mixture to be combined with different fragrance types (not according to the invention) as well as optionally further components of a perfume oil, to produce particularly advantageous fragrance mixtures or to additionally perfume products with any desired scent.

In a preferred embodiment according to the invention, the composition or product is a home care, personal care or pet care composition or product. Preferred personal care compositions/products are cosmetic products and intimate care products, in particular sanitary napkins, diapers, lotion, shampoo, incontinence pads. Preferred home care compositions/products are toilet care products, for example rim blocks, APC Cleaner, Air Care. Preferred pet care compositions/products are animal litter and pet shampoo.

A "fragrance raw material" according to the present invention is a raw material which can be used for fragrance composition as well as perfume oils. Fragrance raw materials can be of synthetic or natural origin. As said above, they can have a pleasant smell which can be easily combined with other fragrance raw materials according to the invention or fragrance raw materials not according to the invention to create advantageous fragrance mixtures or perfume oils.

In a preferred embodiment according to the invention the fragrance raw materials are selected from fragrance raw materials having a molecular weight between 95 to 250 g/mol and essential oils. Preferably, the fragrance raw materials having a molecular weight between 95 to 250 g/mol are selected from acetal, alcohols, hydrocarbon, aldehydes, ketones, ethers, esters, lactone and phenol. More preferably, the fragrances having a molecular weight between 95 to 250 g/mol are selected from the group consisting of Jasmol, cis-1-(1-Ethoxyethoxy)-3-hexene (leaf acetal), 3,7-Dimethylocta-2,6-dien-1-ol, Cinnamyl alcohol, 4-Methyl-2-(2-methylpropyl)oxan-4-ol, Trans-2-Hexenol, Cis-3-Hexenol, 2-Benzylheptanol, Geraniol, Furaneol, Citral, 3-(4-Tert-butylphenyl)butanal, 3-(4-Ethylphenyl)-2,2-dimethylpropanal, 3-(2-Ethylphenyl)-2,2-dimethylpropanal, Aldehyde C12, Trans-2-Hexenal, (±)-2,6-Dimethyl-5-heptenal, (Z,E)-2,4-dimethyl cyclohex-3-ene-1-carbaldehyde, Cyclamen aldehyde, (Z,E)-2,4-dimethyl cyclohex-3-ene-1-carbaldehyde (68039-49-6; 68737-61-1; Ligustral, Tripalal, Cyclal C, Vertocitral) Alpha-hexyl cinnamaldehyde, (2E)-3,7-Dimethylocta-2,6-dienal, 3-(4-Ethylphenyl)-2,2-dimethylpropanal, Helional, Cis-3-hexenyl hexanoate, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate , 1,6-Dihydro-neocarveol Cis-3-hexenyl propionate, Cis-3-hexenyl isobutyrate, Cis-3-hexenyl salicylate, Nerolin Yara Yara, 5-Ethyl-3-hydroxy-4methyl-2(5*H*)-furanon (698-10-2), Terpinene gamma, Ocimene, Beta Ionone,
N-methyl ionone, Methyl heptanone, Alpha Damascone, Alpha-methyl ionone, Methyl cyclopentenolone, 2-Methyl-5-propyl-2-Cyclohexenone, Beta-damascenone, 1-(5,5-Dimethyl-1-cyclohexenyl)pent-4-en-1-one, Dimethyl benzofuranone and Eugenol.

In a more preferred embodiment according to the invention, the fragrances having a molecular weight between

95 to 250 g/mol are selected from the group consisting of cis-1-(1-Ethoxyethoxy)-3-hexene (leaf acetal), Cis-3-Hexenol, 2-Benzylheptanol, Geraniol, Furaneol, Cyclamen aldehyde, (Z,E)-2,4-dimethyl cyclohex-3-ene-1-carbaldehyde (68039-49-6; 68737-61-1; Ligustral, Tripalal, Cyclal C, Vertocitral) Alpha-hexyl cinnamaldehyde, (2E)-3,7-Dimethylocta-2,6-dienal, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol, Cis-3-hexenyl propionate, Cis-3-hexenyl isobutyrate, Cis-3-hexenyl salicylate, 5-Ethyl-3-hydroxy-4methyl-2(5*H*)-furanon (698-10-2),, Terpinene gamma, Ocimene, Methyl heptanone, Alpha Damascone, Alpha-methyl ionone, Methyl cyclopentenolone, 2-Methyl-5-propyl-2-Cyclohexenone, Beta-damascenone, 1-(5,5-Dimethyl-1-cyclohexenyl)pent-4-en-1-one, Dimethyl benzofuranone and Eugenol.

In an even more preferred embodiment according to the invention, the fragrances having a molecular weight between 95 to 250 g/mol are selected from the group consisting of cis-1-(1-Ethoxyethoxy)-3-hexene (leaf acetal), 2-Benzylheptanol, Geraniol, Furaneol, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol, Cis-3-hexenyl propionate, Cis-3-hexenyl isobutyrate, Cis-3-hexenyl salicylate, Ocimene, Alpha-methyl ionone, Methyl cyclopentenolone, 2-Methyl-5-propyl-2-Cyclohexenone, Beta-damascenone, 1-(5,5-Dimethyl-1-cyclohexenyl)pent-4-en-1-one, Dimethyl benzofuranone and Eugenol.

Preferably, the essential oil according to the present invention comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli aclohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta).

Preferably, the present invention refers to the use of a mixture consisting of (a) at least one fragrance raw material having a molecular weight between 95 to 250 g/mol which is selected from the group consisting of cis-1-(1-Ethoxyethoxy)-3-hexene (leaf acetal), 2-Benzylheptanol, Geraniol, Furaneol, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol, Cis-3-hexenyl propionate, Cis-3-hexenyl isobutyrate, Cis-3-hexenyl salicylate, Ocimene, Alpha-methyl ionone, Methyl cyclopentenolone, 2-Methyl-5-propyl-2-Cyclohexenone" Beta-damascenone, 1-(5,5-Dimethyl-1-cyclohexenyl)pent-4-en-1-one, Dimethyl benzofuranone and Eugenol; and (b) EDTA for inhibiting urease.

Preferably, the present invention refers to the use of a mixture consisting of (a) at least one fragrance raw material having a molecular weight between 95 to 225 g/mol which is selected from the group consisting of cis-1-(1-Ethoxyethoxy)-3-hexene (leaf acetal), 2-Benzylheptanol, Geraniol, Furaneol, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol, Cis-3-hexenyl propionate, Cis-3-hexenyl isobutyrate, Cis-3-hexenyl salicylate, Ocimene, Alpha-methyl ionone, Methyl cyclopentenolone, 2-Methyl-5-propyl-2-Cyclohexenone" Beta-damascenone, 1-(5,5-Dimethyl-1-cyclohexenyl)pent-4-en-1-one, Dimethyl benzofuranone and Eugenol; and (b) NBPT for inhibiting urease.

Preferably, the present invention refers to the use of a mixture consisting of

(a) at least one essential oil, wherein the essential oil comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli aclohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta). and

(b) EDTA

for inhibiting urease.

Preferably, the present invention refers to the use of a mixture consisting of

(a) at least one essential oil, wherein the essential oil comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli aclohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta); and

(b) NBPT

for inhibiting urease.

Preferably, the present invention refers to the use of a mixture consisting of

(a) at least one fragrance having a molecular weight between 95 to 250 g/mol are selected from the group consisting of cis-1-(1-Ethoxyethoxy)-3-hexene (leaf acetal), 2-Benzylheptanol, Geraniol, Furaneol, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol, Cis-3-hexenyl propionate, Cis-3-hexenyl isobutyrate, Cis-3-hexenyl salicylate, Ocimene, Alpha-methyl ionone, Methyl cyclopentenolone, 2-Methyl-5-propyl-2-Cyclohexenone" Beta-damascenone, 1-(5,5-Dimethyl-1-cyclohexenyl)pent-4-en-1-one, Dimethyl benzofuranone and Eugenol; and at least one essential oil selected, wherein the essential oil comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli aclohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta).; and

(b) EDTA

Preferably, the present invention refers to the use of a mixture consisting of

(a) at least one fragrance having a molecular weight between 95 to 250 g/mol are selected from the group consisting of cis-1-(1-Ethoxyethoxy)-3-hexene (leaf acetal), 2-Benzylheptanol, Geraniol, Furaneol, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol, Cis-3-hexenyl propionate, Cis-3-hexenyl isobutyrate, Cis-3-hexenyl salicylate, Ocimene, Alpha-methyl ionone, Methyl cyclopentenolone, 2-Methyl-5-propyl-2-Cyclohexenone" Beta-damascenone, 1-(5,5-Dimethyl-1-cyclohexenyl)pent-4-en-1-one, Dimethyl benzofuranone and Eugenol; and at least one essential oil, wherein the essential oil comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli aclohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta).; and

(b) NBPT

Preferably, the present invention refers to the use of a mixture consisting of

(a) at least one fragrance having a molecular weight between 95 to 250 g/mol are selected from the group consisting of cis-1-(1-Ethoxyethoxy)-3-hexene (leaf acetal), 2-Benzylheptanol, Geraniol, Furaneol, 3-(4-Ethylphenyl)-2,2-dimethylpropanal, Helional, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate, 1,6-Dihydro-neo-carveol Cis-3-hexenyl propionate, Cis-3-hexenyl isobutyrate, Cis-3-hexenyl salicylate, Ocimene, Alpha-methyl ionone, Methyl cyclopentenolone, 2-Methyl-5-propyl-2-Cyclohexenone" Beta-damascenone, 1-(5,5-Dimethyl-1-cyclohexenyl)pent-4-en-1-one, Dimethyl benzofuranone and Eugenol; and at least one essential oil, wherein the essential oil comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli aclohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta).; and

(b) EDTA and NBPT

In a preferred embodiment according to the invention, the amount of the at least one fragrance raw material is of from 0.1 to 75 weight percent, calculated on the total amount of the mixture. Preferably, the amount of EDTA is of from 0.1 to 50 weight percent, calculated on the total amount of the mixture. Preferably, the amount of NBPT is of from 0.01 to 25 weight percent, calculated on the total amount of the mixture.

In a preferred embodiment according to the invention, the ratio of the at least one fragrance raw material to EDTA and/or NBPT is 1:5 to 5:1.

It goes without saying that one or more fragrance raw materials can be used to inhibit urease activity. Therefore, for example one, two, three, four, five, six, seven, eight, nine, ten or even twenty to thirty fragrance raw materials according to the present invention can be used to inhibit urease activity in the mixture according to the invention.

Another embodiment of the present invention relates to a personal care composition comprising the mixture consisting of

(a) at least one fragrance raw material and

(b) EDTA and/or NBPT.

Another embodiment of the present invention relates to a home care composition comprising the mixture consisting of

(a) at least one fragrance raw material and

(b) EDTA and/or NBPT.

Another embodiment of the present invention relates to a pet care composition comprising the mixture consisting of

(a) at least one fragrance raw material and

(b) EDTA and/or NBPT.

Preferably, the mixture is used in an amount of from 0.01 to 10 weight percent when added to a composition or product, calculated on the total weight of the composition or product. More preferably, the mixture is used in an amount of from 0.05 to 5 weight percent; more preferably in an amount of from 0.05 to 1 weight percent and most preferably in an amount of from 0.1 to 1 weight percent; all weight percentages calculated on the total weight of the composition or product. When the mixture according to the present invention is used in rim blocks, it is preferably used in an amount of from 0.01 to 10 weight percent, more preferably in an amount of from 1 to 8 weight percent, more preferably in an amount of from 2 to 6 weight percent, even more preferably in an amount of from 3,5 to 4.5 weight percent, calculated on the total weight of the composition or product.

Particularly advantageous is a use according to the invention in which the ratio of the total mass of the mixture to the total mass of at least one precursor of ammonia is from 2:1 to 1:5, more preferably from 1:1 to 1:3.

A "precursor" according to the present invention is a compound that participates in a chemical reaction that produces another compound. In the present case, a "precursor of ammonia" is a compound that participates in a chemical reaction that produces ammonia. In one embodiment according to the invention, the precursor of ammonia is urea.

Thus, in a preferred embodiment according to the invention, the mixture is used as urease inhibitor in a composition or product, in particular in home care, personal care and pet care compositions/products, together with at least one precursor of ammonia. Therefore, it is possible, when getting in contact with, for example, bacterial ureases, to completely, but at least partially, prevent the formation of ammonia by means of the mixture of the present invention.

It goes without saying that one or more fragrance raw materials can be used in the mixture to inhibit urease activity. Therefore, for example one, two, three, four, five, six, seven, eight, nine, ten fragrances having a molecular weight between 95 to 250 g/mol can be used to inhibit urease activity in a home care, personal care or pet care composition/-product.

Examples of other fragrances which can in principle be advantageously used as a further component together

with the mixture, can be found, for example, in S. Arctander, Perfume and Flavor Chemicals, Vol. I and II, Montclair, N. J., 1969, Selbstverlag oder H. Surburg, J. Panten, Common Fragrance and Flavor Materials, 5th Ed., Wiley-VCH, Weinheim 2006.

In some embodiments according to the invention, the mixture of the invention can be combined with further fragrances or solvents. The mixture according to the invention can be in liquid form, undiluted or diluted with a solvent and is advantageously used for perfuming. Preferred solvents for this purpose are ethanol, isopropanol, diethylene glycol monoethyl ether, glycerol, propylene glycol, 1 ,2-butylene glycol, dipropylene glycol, diethyl phthalate, triethyl citrate, isopropyl myristate, triacetin, 2,2-dimethyl-1,3-dioxolane-4-methanol and diacetin.

Furthermore, the mixture according to the invention may be adsorbed on a carrier that provides both fine distribution of the fragrance raw materials in the product and controlled release upon application. Such carriers may be porous inorganic materials such as light sulfate, silica gels, zeolites, gypsums, clays, clay granules, aerated concrete, etc., or organic materials such as woods, cellulose-based materials, sugars, dextrins (e.g., maltodextrin), or plastics such as PVC, polyvinyl acetates, or polyurethanes. T

The mixture according to the invention may also be microencapsulated, spray-dried, as inclusion complexes, or as extrusion products, and may be added in this form, for example, to a product to be perfumed.

If necessary, the properties of the compositions can be further optimized by so-called "coating" with suitable materials with a view to more targeted fragrance release, for which purpose waxy plastics such as polyvinyl alcohol are preferably used.

Preferably, the personal care, home care or pet care composition according to the invention do not contain any further urease inhibitor

Other preferred embodiments and definitions which have been described above also apply, of course, to the personal care, home care and pet care composition/product according to the invention.

A further embodiment according to the invention refers to a method for inhibiting urease activity in a personal care, home care or pet care composition, wherein a mixture consisting of

(a) at least one fragrance raw material and

(b) EDTA and/or NBPT

is added to the personal care, home care or pet care composition

Preferably, the mixture is added in an amount of from 0.01 to 10 weight percent, calculated on the total weight of the personal care, home care or pet care composition. More preferably, the mixture is added in an amount of from 0.05 to 5 weight percent, calculated on the total weight of the personal care, home care or pet care composition. Even more preferably, the mixture is added in an amount of from 0.05 to 1 weight percent, calculated on the total weight of the personal care, home care or pet care composition. When the mixture according to the present invention is added to rim blocks, it is preferably used in an amount of from 0.01 to 10 weight percent, more preferably in an amount of from 1 to 8 weight percent, more preferably in an amount of from 2 to 6 weight percent, even more preferably in an amount of from 3,5 to 4.5 weight percent, calculated on the total weight of the composition or product.

The above-mentioned preferred embodiments and definitions of course also apply to the method according to the present invention.

In the following, embodiments of the invention are further characterized.

If not indicated otherwise, all amounts are indicated in weight percent.

## EXAMPLES

### Example 1

*Screening of Urease Inhibition*

**Materials:**

Stock Solutions:

PBS 2x: PBS 2x solution is prepared by dilution of PBS 10x. To this diluted solution ~0,5 mg of phenol red is added. pH is set to 5 - 6 with HCl (37%). The optimal color of the solution is yellow.

- Urea: 10 g/L stock solution of urea is prepared in distilled water.

- MasterMix - Stock of PBS and urea: 12 mL 3x PBS + 2,4 ml urea stock solution + 6 ml distilled water.
- Urease: Urease solution is prepared with the activity of ~20 U/mL in distilled water, using *Urease ous Canavalia ensiformis (Jack bean) - Sigma Aldrich (500-800 U/mL).*
- FRM / NBPT (as PS): In the case of a liquid, 80µL of the raw material of interest are placed in a 5mL bottle using a micropipette. It is diluted using 4mL of DMSO. This solvent has been chosen as it does not inhibit the urease activity or the ammonia formation. In the case of a solid, 80µg of the raw material of interest are weighed with a precision scale and placed in a 5mL bottle. It is diluted using 4mL of DMSO.

**Test method:**

The test is performed in optical plate reader with 96-well plates. Ammonia formation with the combination of urea and urease in case of no inhibitor addition, alters the pH of the solution, which changes the color yellow to pink. The color change, as indirect indicator for ammonia formation, is then detected and measured by the optic lenses and the output of optical density (560 nm) vs time graph is generated.

Preparation:

1. 10µl of fragrance raw material (FRM), NBPT (PS), and DMSO (NS) is added to wells.
2. Following, the urease is added with the amount of 20µl (distilled water for the blank samples).
3. 170 µl of MasterMix, containing urea substrate, is added to the mixture in the well, and shaked well

The reaction may start rapidly, the plate should be inserted to the plate reader before the color change.

Protocol procedure:

The test is conducted for 1 hour time scale under the set temperature of 24°C. Read is performed as kinetic at 560 nm. Interval measurement is 1min.

**Table 1: Final concentrations in the assay:**

| | |
|---|---|
| FRM/NPBT | 0,1% (FRMs) up to 0,5% (FRA) |
| urea | 1g/L |
| urease | 2 U/mL |
| PBS | 1x |
| total volume | 200 µL |

Inhibition value:

The rate of reaction is derived from the color change (Δ Absorbance at 560nm) over time:

$$\Delta\, Absorbance = \frac{Absorbance\ t_1 - Absorbance\ t_0}{t_1 - t_0}$$

The inhibition efficacy is then calculated as follows:

$$I = 1 - \left(\frac{\Delta\, Absorbance\ of\ sample}{\Delta\, Absorbance\ of\ negative\ control}\right)$$

**Identification of positive hits**

**Fig. 1** represents a typical result from the screening assay. The corresponding calculations are shown in table 2. In this case, the fragrance raw material shows a significant inhibition of the enzyme urease.

**Table 2: Results screening assay**

| Samples | Δ Absorbance at 1h (560nm) | Inhibition |
|---|---|---|
| **Negative control, w/o additive** | 0,61 | 0 |
| **Positive control, with inhibitor (NBPT)** | 0,00 | 1,00 |
| **Sample with fragrance raw material** | 0,07 | 0,89 |

**Positive hits**

A total of 56 highly active compounds could be identified through *in vitro* screening. Interestingly, they share common structural features like a molecular weight between 95 to 250 g/mol, are acetal, alcohols, hydrocarbon, aldehydes, ketones, ethers, esters, lactone, phenol.

**Table 3: Highly active compounds**

| Name | functional group | inhibition rate (normed, based on NBPT) |
|---|---|---|
| EUCALYPTOL | ALCOHOL | 1,041 |
| PINEN BETA | HYDROCARBON | 1,027 |
| VETIVEROL | HYDROCARBON | 1,001 |
| FURANEOL | ALCOHOL | 0,997 |
| GERANIAL | ALDEHYDE | 0,992 |
| CITRONELLOL | ALCOHOL | 0,982 |
| GERMACRENE D | HYDROCARBON | 0,981 |
| HELIONAL | ALDEHYDE | 0,975 |
| PATCHOULI ALCOHOL | ALCOHOL | 0,972 |
| LINALOOL | ALCOHOL | 0,969 |
| FLORALOZONE | ALDEHYDE | 0,954 |
| DYNASCONE | KETONE | 0,928 |
| FURAMINTON/TONKALACTONE™ | LACTONE | 0,889 |
| DAMASCENONE TOTAL | KETONE | 0,887 |
| HEXENYL SALICYLATE CIS-3 | ESTER | 0,885 |
| HEXENYL ISOBUTYRATE CIS-3 | ESTER | 0,883 |
| LIMONENE D nat | HYDROCARBON | 0,866 |
| HEXENYL PROPIONATE CIS-3 | ESTER | 0,859 |
| 2-METHYL-5-PROPYL-2-CYCLOHEXENONE | KETONE | 0,847 |

(continued)

| Name | functional group | inhibition rate (normed, based on NBPT) |
|---|---|---|
| EUGENOL NAT. | PHENOL | 0,846 |
| OCIMENE | HYDROCARBON | 0,836 |
| GERANIOL 60 B | ALCOHOL | 0,834 |
| CARVYL ACETATE CIS- | ESTER | 0,828 |
| JASMOL | ALCOHOL | 0,817 |
| CETALEAF | ACETAL | 0,812 |
| METHYL CYCLOPENTENOLONE-3,2,2 NAT. | KETONE | 0,808 |
| HEXENYL ISOVALERATE CIS-3 | ESTER | 0,804 |
| HEXENYL ACETATE CIS-3 | ESTER | 0,779 |
| HEXENYL BUTYRATE CIS-3 | ESTER | 0,779 |
| METHYL IONONE ALPHA / IFF | KETONE | 0,767 |
| CAREN DELTA-3 | HYDROCARBON | 0,728 |
| HIMACHALEN BETA | HYDROCARBON | 0,725 |
| CITRAL FF | ALDEHYDE | 0,701 |
| DAMASCONE ALPHA | KETONE | 0,676 |
| HEXENOL CIS-3 | ALCOHOL | 0,640 |
| HEXYL CINNAMIC ALDEHYDE ALPHA | ALDEHYDE | 0,626 |
| METHYL ETHYL HYDROXYFURANONE | HETEROCYCLIC | 0,611 |
| VERTOCITRAL | ALDEHYDE | 0,559 |
| METHYL HEPTENONE-6,5,2 | KETONE | 0,549 |
| CYCLAMEN ALDEHYDE | ALDEHYDE | 0,543 |
| TERPINENE GAMMA | HYDROCARBON | 0,523 |
| ISORALDEINE 70 | KETONE | 0,500 |
| IONONE BETA | KETONE | 0,495 |
| LIGUSTRAL | ALDEHYDE | 0,482 |
| HEXENOL TRANS-2 | ALCOHOL | 0,455 |
| HEXENYL CAPROATE CIS-3 | ESTER | 0,449 |
| FLOROSA | ALCOHOL | 0,442 |
| CINNAMIC ALCOHOL | ALCOHOL | 0,430 |
| MELONAL | ALDEHYDE | 0,429 |
| HEXENAL TRANS-2 | ALDEHYDE | 0,381 |
| ALDEHYDE C12 MNA | ALDEHYDE | 0,377 |
| FLORAZON | ALDEHYDE | 0,371 |
| LILIAL | ALDEHYDE | 0,347 |
| NEROLIN YARA YARA CRIST. | ETHER | 0,325 |
| PINEN ALPHA | HYDROCARBON | 0,322 |
| CITRAL 95 | ALDEHYDE | 0,322 |
| NEROL 900 | ALCOHOL | 0,311 |
| EUGENOL NAT. | ALCOHOL | 0,264 |

**Raw material screening**

Fragrance raw materials are used in a concentration of 0.1% based on total FRA amount of 4%. Reduction of ammonia formation is based on urease inhibition plus additional chemical reaction over time to remove formed ammonia if urease is not fully inhibited. **Figures 2 and 3** show the results.

**Accord screening**

Concentration accords: total fragrance composition concentration in rim block is 4%. Accord is added up to 1.2% of total FRA amount, meaning 30% of total FRA consists of accord. Remaining percentage is filled up with DPG. **Figures 4,**

**5 and 6** show the results.

**Accord composition**

| **Accord AB** | |
|---|---|
| **name** | **amount** |
| DYNASCONE | 5 |
| CITRAL FF | 5 |
| EUCALYPTOL | 50 |
| HEXENYLACETAT CIS-3 | 2,5 |
| MELONAL | 1 |
| LIMONEN | 906,5 |
| VERTOCITRAL | 30 |
| **total** | 1000 |

| **Accord AC** | |
|---|---|
| **name** | **amount** |
| CITRAL FF | 40 |
| DYNASCONE | 4 |
| GERANIAL | 10 |
| OCIMEN | 5 |
| LIMONEN | 906 |
| TERPINEN GAMMA | 35 |
| **total** | 1000 |

| **Accord AD** | |
|---|---|
| **name** | **amount** |
| FURAMINTON/TONKALACTONE™ | 5 |
| LINALOOL | 978 |
| MELONAL | 10 |
| OCIMEN MIXTURE OF Cis-Carvyl acetate, Trans-Carvyl acetate, 1,6-Dihydro-carvyl acetate, | 5 |
| 1,6-Dihydro-neo-carveol | 2 |
| **total** | 1000 |

**Example 2**

*Rim blocks*

In 250 mL Duran bottles, 47 mL urea solution (50 g/L, pH: 5.0) is added. Then 500 mg rim block mass, containing FRA or EDTA or NBPT is added. The reaction is started by adding 500 $\mu$L urease (500-800 U/mL) under stirring (80 rpm). The rim block mass should not touch the stirring fish. The ammonia concentration is determined analytically using a sensitive ammonia device. As measuring times 15min - 2h - 4h - 6h- 24h - 26h and 30 h are chosen. The measurement is stopped as soon as the steady state is reached.

For negative control the rim block mass contains DPG as filler (The efficiency of DPG was tested beforehand - no inhibition observable on analytical scale assay and in rim block base)

For positive Control 0.1% Neofresh Ammonia (contains 0.025% NBPT) in incorporated into the rim block with 3.9% DPG

Test samples contain between 0.1% - 1% EDTA or 0.1% - 4% FRMs. Each time when 4% amount of material is not

reached the remaining amount is filled with DPG.

Accords consisting of best performer FRMs from enzymatic assay are incorporated into rim block base in different concentrations from 0.1% -4% to prove concentration dependent inhibition properties.

**Claims**

1. Use of a mixture consisting of

 (a) at least one fragrance raw material and
 (b) EDTA and/or NBPT

 for inhibiting urease.

2. The use according to claim 1, wherein the fragrance raw material is selected from fragrance raw materials having a molecular weight between 95 to 250 g/mol and essential oils.

3. The use according to claim 1 and/or 2, wherein the fragrance raw materials having a molecular weight between 95 to 250 g/mol are selected from the group consisting of cis-1-(1-Ethoxyethoxy)-3-hexene (leaf acetal), 3,7-Dimethylocta-2,6-dien-1-ol, Cinnamyl alcohol, 4-Methyl-2-(2-methylpropyl)oxan-4-ol, Trans-2-Hexenol, Cis-3-Hexenol, 2-Benzylheptanol, Geraniol, Furaneol, Citral, 3-(4-Tert-butylphenyl)butanal, 3-(4-Ethylphenyl)-2,2-dimethylpropanal, 3-(2-Ethylphenyl)-2,2-dimethylpropanal, Aldehyde C12, Trans-2-Hexenal, (±)-2,6-Dimethyl-5-heptenal, (Z,E)-2,4-dimethyl cyclohex-3-ene-1-carbaldehyde, Cyclamen aldehyde, (Z,E)-2,4-dimethyl cyclohex-3-ene-1-carbaldehyde (68039-49-6; 68737-61-1; Ligustral, Tripalal, Cyclal C, Vertocitral), Alpha-hexyl cinnamaldehyde, (2E)-3,7-Dimethylocta-2,6-dienal, 3-(4-Ethylphenyl)-2,2-dimethylpropanal, Helional, Cis-3-hexenyl hexanoate, Cis-3-hexenyl butyrate, Cis-3-hexenyl acetate, Cis-3-Hexenyl isovalerate, Cis-Carvyl acetate, Trans-Carvyl acetate, 1,6-Dihydro-carvyl acetate , 1,6-Dihydro-neo-carveol, Cis-3-hexenyl propionate, Cis-3-hexenyl isobutyrate, Cis-3-hexenyl salicylate, Nerolin Yara Yara, 5-Ethyl-3-hydroxy-4methyl-2(5*H*)-furanon (698-10-2), Terpinene gamma, Ocimene, Beta Ionone, N-methyl ionone, Methyl heptanone, Alpha Damascone, Alpha-methyl ionone, Methyl cyclopentenolone, 2-Methyl-5-propyl-2-Cyclohexenone" Beta-damascenone, 1-(5,5-Dimethyl-1-cyclohexenyl)pent-4-en-1-one, Dimethyl benzofuranone and Eugenol.

4. The use according to at least one of the preceding claims, wherein the essential oil comprises at least one of the following compounds as main ingredients: Eucalyptol, Geranial, Citronellol, Linalool, alpha-Pinen (80-65-8), beta-Pinen (127-91-3), Eugenol, (1E,6E,8S)-1-methyl-5-methylidene-8-propan-2-ylcyclodeca-1,6-diene (23986-74-5, Germacren D), 4,8-dimethyl-2-propan-2-ylidene-3,3a,4,5,6,8a-hexahydro-1H-azulen-6-ol (68129-81-7, Vetiverol), (1R,3R,6S)-2,2,6,8-Tetramethyl-tricyclo[5.3.1.03,8]undec-3-ol (5986-55-0, Patchouli alcohol), p-Mentha-1,8-dien (5989-27-5, Limonen D), (+)-3-Caren, (1S)-3,7,7-Trimethyl-bicyclo[4.1.0]hept-3-en (498-15-7, delta-3-Caren), 2,5,9,9-tetramethyl-3,4,6,7,8,9a-hexahydrobenzo[7]annulene (1461-03-6, Himachalen beta).

5. The use according to at least one of the preceding claims, wherein the amount of the at least one fragrance raw material is of from 0.1 to 75 weight percent, calculated on the total amount of the mixture.

6. The use according to at least one of the preceding claims, wherein the amount of EDTA is of from 0.1 to 50 weight percent, calculated on the total amount of the mixture.

7. The use according to at least one of the preceding claims, wherein the amount of NBPT is of from 0.01 to 25 weight percent, calculated on the total amount of the mixture.

8. The use according to at least one of the preceding claims, wherein the ratio of the at least one fragrance raw material to EDTA and/or NBPT is 1:5 to 5:1.

9. - A personal care composition comprising the mixture consisting of

 (a) at least one fragrance raw material and
 (b) EDTA and/or NBPT.

10. A home care composition comprising the mixture consisting of

(a) at least one fragrance raw material and
(b) EDTA and/or NBPT.

**11.** A pet care composition comprising the mixture consisting of

(a) at least one fragrance raw material and
(b) EDTA and/or NBPT.

**12.** The personal care, home care or pet care composition according to claims 9, 10 or 11, wherein the composition does not contain any further urease inhibitor.

**13.** The personal care, home care or pet care composition according to claims 9, 10, 11 and/or 12, wherein the fragrance raw material is selected from fragrance raw materials having a molecular weight between 95 to 250 g/mol and essential oils.

**14.** A method for inhibiting urease activity in a personal care, home care or pet care composition, wherein a mixture consisting of

(a) at least one fragrance raw material and
(b) EDTA and/or NBPT

is added to the personal care, home care or pet care composition

**15.** The method according to claim 14, wherein the mixture is added in an amount of from 0.01 to 10 weight percent to the personal care, home care or pet care composition, calculated on the total weight of the personal care, home care or pet care composition.

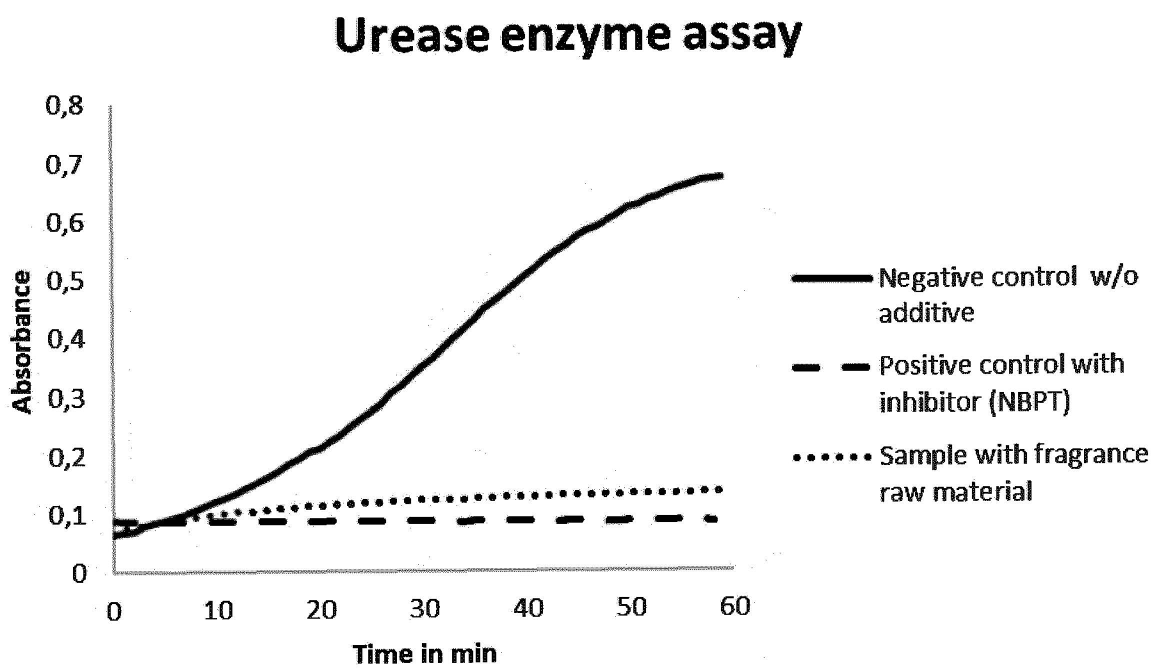
Urease enzyme assay
0,8
0,7
0,6
0,5
0,4
0,3
0,2
0,1
0
Absorbance
0
10
20
30
40
50
60
Time in min
Negative control w/o additive
Positive control with inhibitor (NBPT)
Sample with fragrance raw material

Figure (1/7)

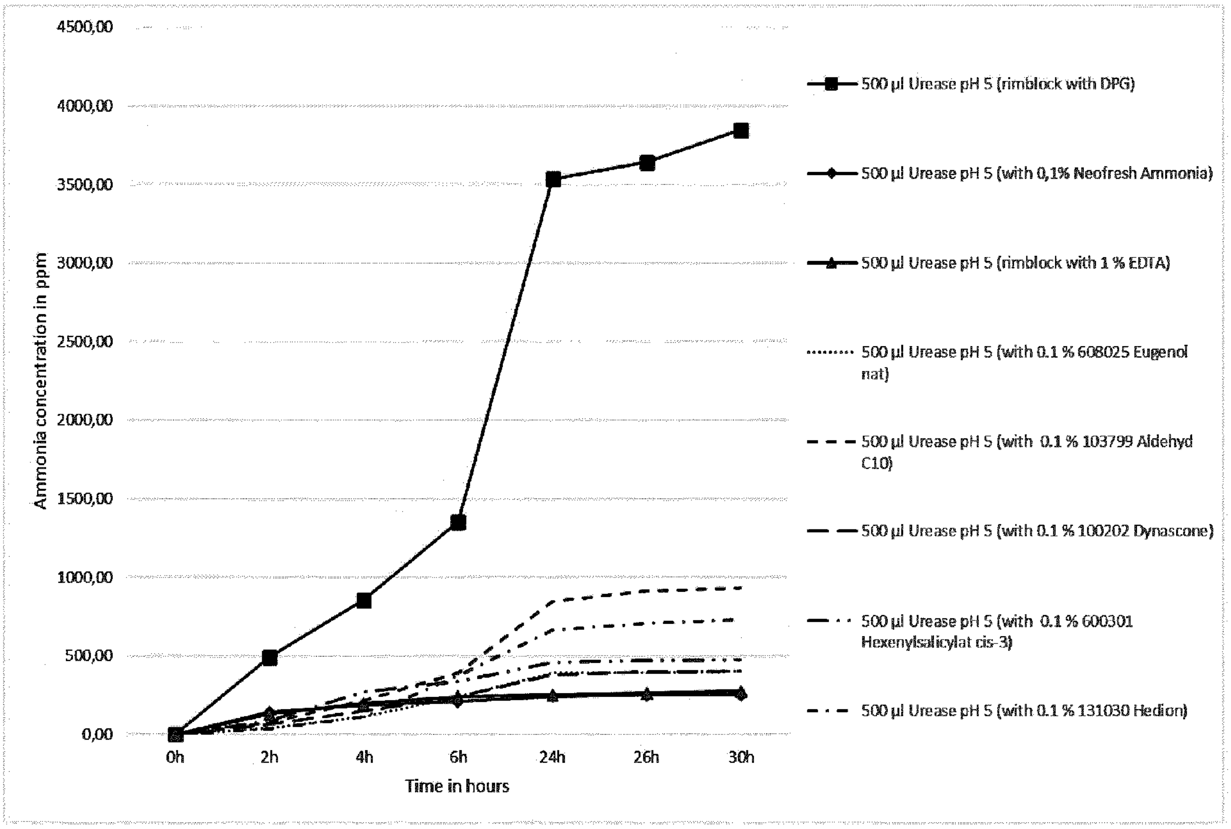
4500,00
4000,00
3500,00
3000,00
2500,00
2000,00
1500,00
1000,00
500,00
0,00
Ammonia concentration in ppm
0h
2h
4h
6h
24h
26h
30h
Time in hours
500 µl Urease pH 5 (rimblock with DPG)
500 µl Urease pH 5 (with 0,1% Neofresh Ammonia)
500 µl Urease pH 5 (rimblock with 1 % EDTA)
500 µl Urease pH 5 (with 0.1 % 608025 Eugenol nat)
500 µl Urease pH 5 (with 0.1 % 103799 Aldehyd C10)
500 µl Urease pH 5 (with 0.1 % 100202 Dynascone)
500 µl Urease pH 5 (with 0.1 % 600301 Hexenylsalicylat cis-3)
500 µl Urease pH 5 (with 0.1 % 131030 Hedion)

Figure (2/7)

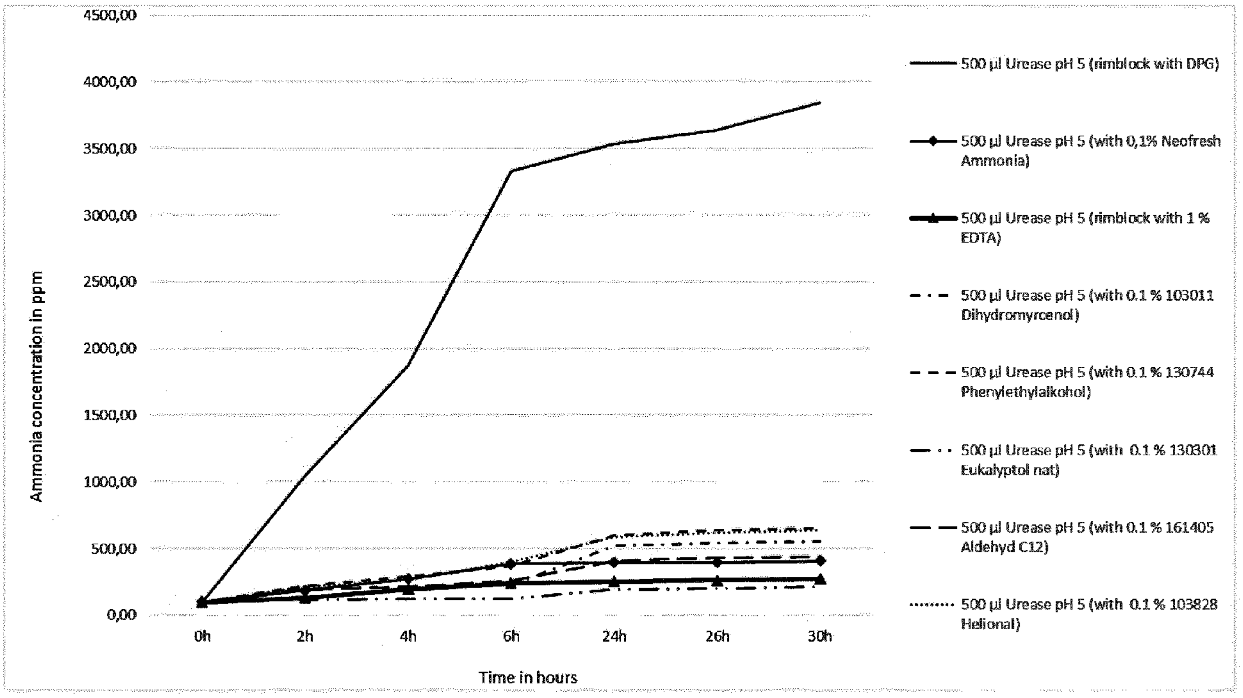
4500,00
4000,00
3500,00
3000,00
2500,00
2000,00
1500,00
1000,00
500,00
0,00
Ammonia concentration in ppm
0h
2h
4h
6h
24h
26h
30h
Time in hours
500 µl Urease pH 5 (rimblock with DPG)
500 µl Urease pH 5 (with 0,1% Neofresh Ammonia)
500 µl Urease pH 5 (rimblock with 1 % EDTA)
500 µl Urease pH 5 (with 0.1 % 103011 Dihydromyrcenol)
500 µl Urease pH 5 (with 0.1 % 130744 Phenylethylalkohol)
500 µl Urease pH 5 (with 0.1 % 130301 Eukalyptol nat)
500 µl Urease pH 5 (with 0.1 % 161405 Aldehyd C12)
500 µl Urease pH 5 (with 0.1 % 103828 Helional)

**Figure (3/7)**

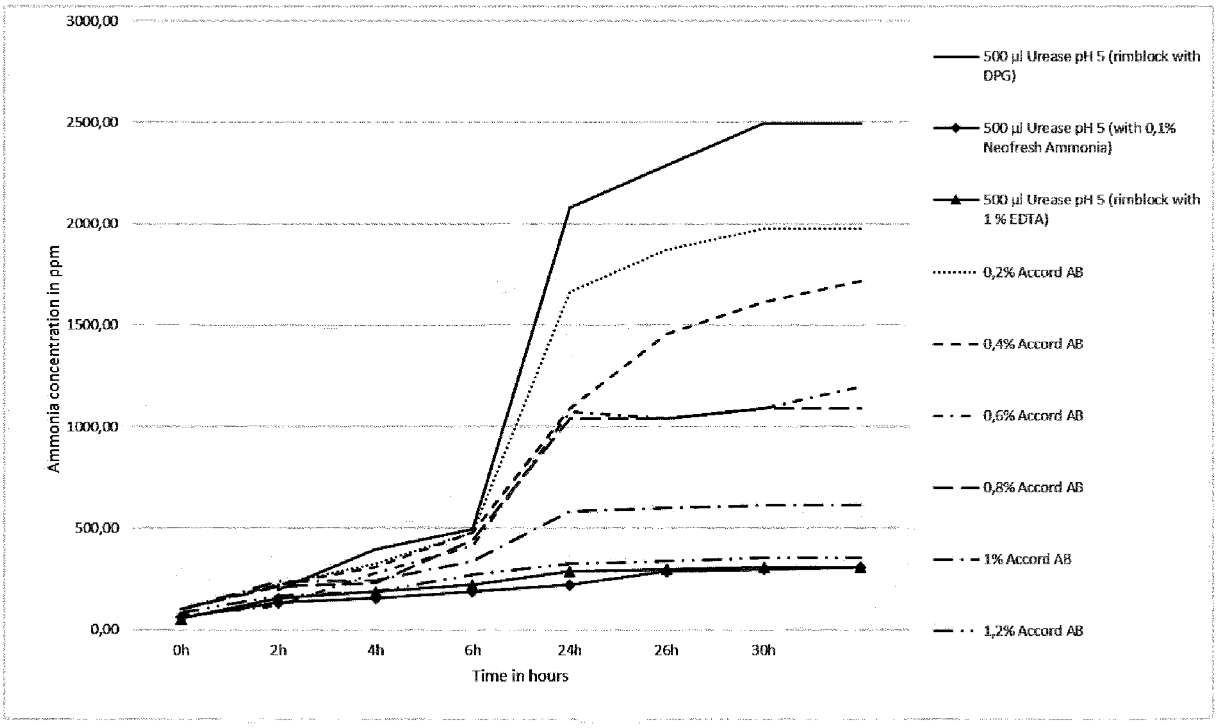
3000,00
2500,00
2000,00
1500,00
1000,00
500,00
0,00
Ammonia concentration in ppm
0h
2h
4h
6h
24h
26h
30h
Time in hours
500 µl Urease pH 5 (rimblock with DPG)
500 µl Urease pH 5 (with 0,1% Neofresh Ammonia)
500 µl Urease pH 5 (rimblock with 1 % EDTA)
0,2% Accord AB
0,4% Accord AB
0,6% Accord AB
0,8% Accord AB
1% Accord AB
1,2% Accord AB

Figure (4/7)

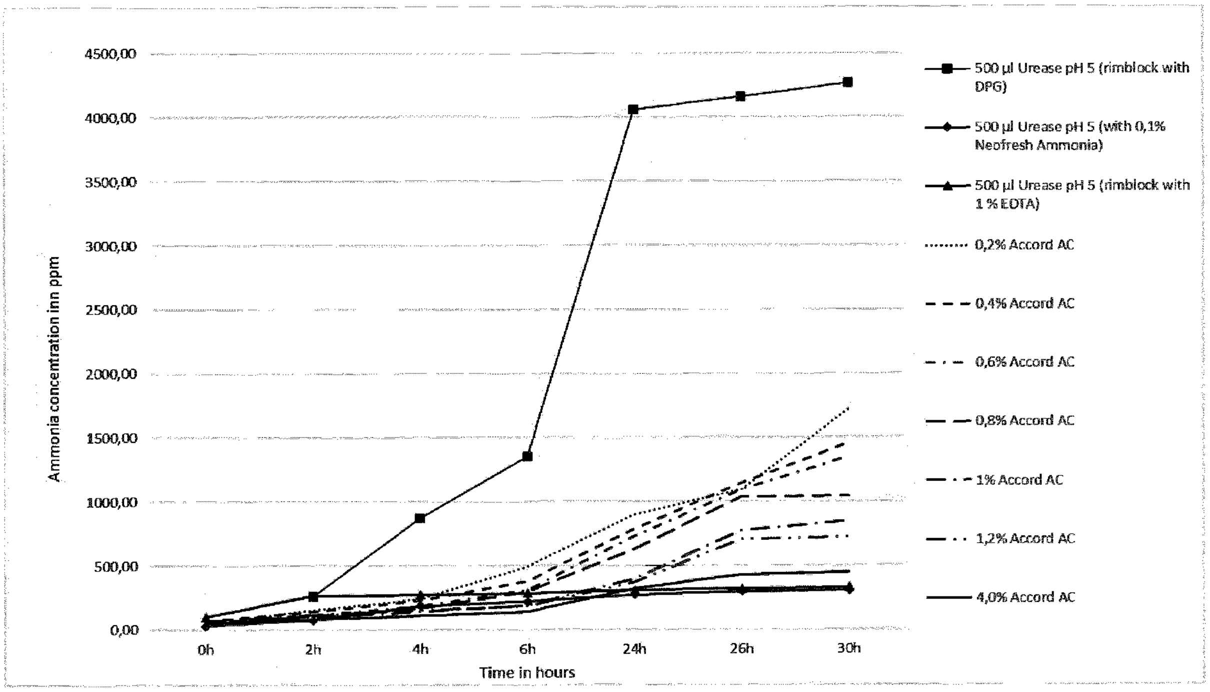
4500,00
4000,00
3500,00
3000,00
2500,00
2000,00
1500,00
1000,00
500,00
0,00
Ammonia concentration inn ppm
0h
2h
4h
6h
24h
26h
30h
Time in hours
500 µl Urease pH 5 (rimblock with DPG)
500 µl Urease pH 5 (with 0,1% Neofresh Ammonia)
500 µl Urease pH 5 (rimblock with 1 % EDTA)
0,2% Accord AC
0,4% Accord AC
0,6% Accord AC
0,8% Accord AC
1% Accord AC
1,2% Accord AC
4,0% Accord AC

**Figure (5/7)**

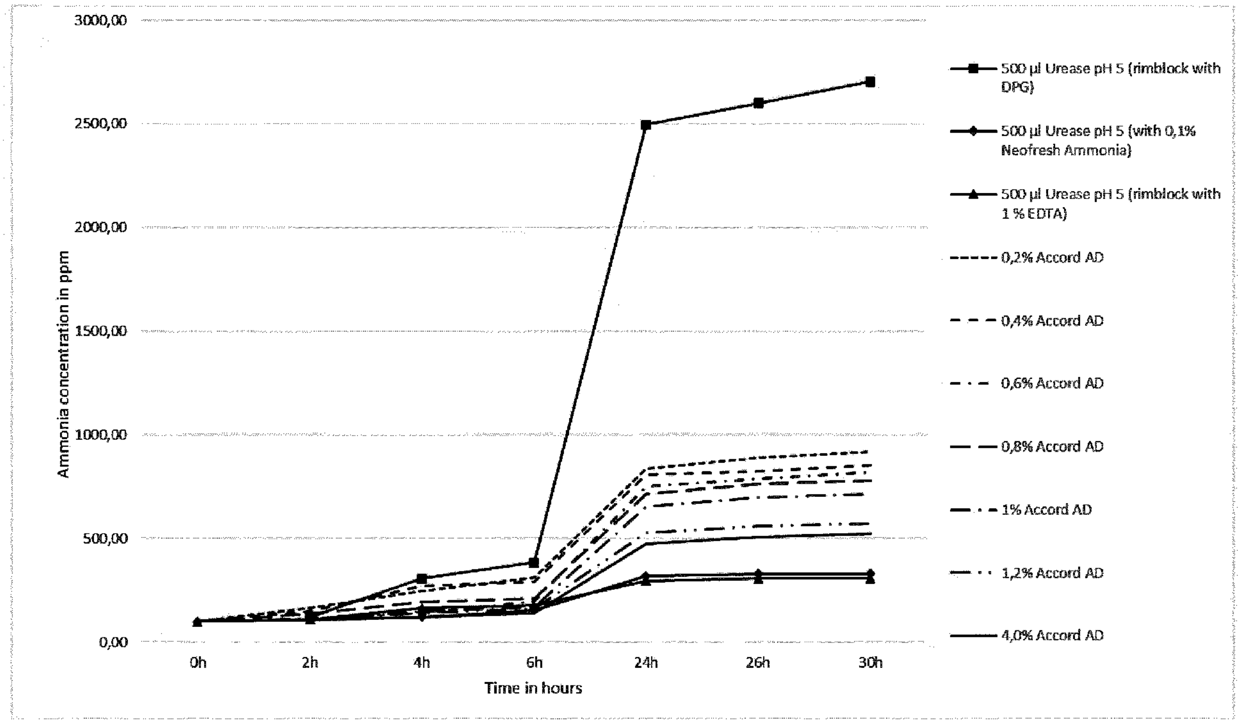
3000,00
2500,00
2000,00
1500,00
1000,00
500,00
0,00
Ammonia concentration in ppm
0h
2h
4h
6h
24h
26h
30h
Time in hours
500 µl Urease pH 5 (rimblock with DPG)
500 µl Urease pH 5 (with 0,1% Neofresh Ammonia)
500 µl Urease pH 5 (rimblock with 1 % EDTA)
0,2% Accord AD
0,4% Accord AD
0,6% Accord AD
0,8% Accord AD
1% Accord AD
1,2% Accord AD
4,0% Accord AD

Figure (6/7)

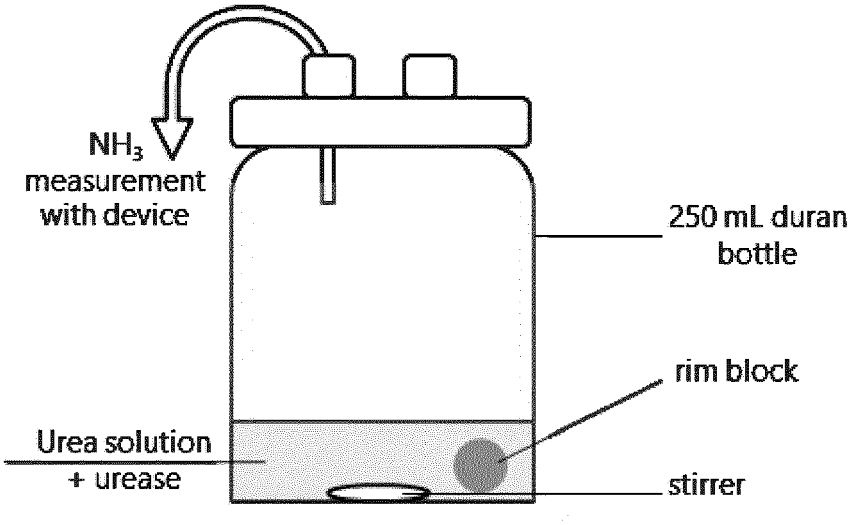
NH3
measurement
with device
250 mL duran
bottle
rim block
Urea solution
+ urease
stirrer

Figure (7/7)

Europäisches Patentamt
European Patent Office
Office européen des brevets

# EUROPEAN SEARCH REPORT

Application Number
EP 23 19 8667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2019/145557 A1 (SPECIALITES PET FOOD [FR]) 1 August 2019 (2019-08-01)<br>* examples 1, 4 * | 1,2,5,7, 8,14<br>3 | INV.<br>A01K1/015<br>A61K8/362<br>A61L9/01<br>A61L15/20<br>A61Q5/02<br>A61Q15/00<br>E03D9/00<br>A61K8/41<br>A61K8/46<br>A61K8/55<br>A61L9/05 |
| X | WO 2015/132267 A1 (ONTEX BVBA [BE]) 11 September 2015 (2015-09-11)<br>* example 1 * | 6,9,12, 13,15 | |
| X<br><br><br><br><br><br>A | DATABASE GNPD [Online]<br>MINTEL;<br>19 May 2009 (2009-05-19),<br>anonymous: "Stain & Odor Eliminator for Pets",<br>XP93134756,<br>Database accession no. 1110590<br>* the whole document * | 11,12<br><br><br><br><br><br>1-10, 13-15 | |
| X | DATABASE GNPD [Online]<br>MINTEL;<br>16 August 2013 (2013-08-16),<br>anonymous: "Liquid Toilet Cleaner",<br>XP93134992,<br>Database accession no. 2148699<br>* abstract * | 10 | **TECHNICAL FIELDS SEARCHED (IPC)**<br>A01K<br>A61L<br>D06Q<br>A61Q<br>E03D<br>A61K |
| Y<br><br><br><br><br><br><br><br><br>A | LUZIA V. MODOLO ET AL: "An overview on the potential of natural products as ureases inhibitors: A review",<br>JOURNAL OF ADVANCED RESEARCH,<br>vol. 6, no. 1, 1 January 2015 (2015-01-01), pages 35-44, XP055252123,<br>AMSTERDAM, NL<br>ISSN: 2090-1232, DOI:<br>10.1016/j.jare.2014.09.001<br>* p.42, left col., 3rd para. * | 3<br><br><br><br><br><br><br><br><br>1,2,4-15 | |

-/--

The present search report has been drawn up for all claims

1

| Place of search | Date of completion of the search | Examiner |
|---|---|---|
| The Hague | 27 February 2024 | Kelly, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 19 8667

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VAREL V.H. ET AL: "Combination of a urease inhibitor and a plant essential oil to control coliform bacteria, odour production and ammonia loss from cattle waste", JOURNAL OF APPLIED MICROBIOLOGY, vol. 102, no. 2, 1 February 2007 (2007-02-01), XP93135155, GB ISSN: 1364-5072, DOI: 10.1111/j.1365-2672.2006.03120.x | 1,4 | |
| A | * p.475, left hand column, results. * ----- | 2,3,5-15 | |

TECHNICAL FIELDS SEARCHED (IPC)

The present search report has been drawn up for all claims

| Place of search | Date of completion of the search | Examiner |
|---|---|---|
| The Hague | 27 February 2024 | Kelly, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO.

EP 23 19 8667

This annex lists the patent family members relating to the patent documents cited in the above-mentioned European search report. The members are as contained in the European Patent Office EDP file on
The European Patent Office is in no way liable for these particulars which are merely given for the purpose of information.

27-02-2024

| Patent document cited in search report | Publication date | Patent family member(s) | Publication date |
|---|---|---|---|
| WO 2019145557 A1 | 01-08-2019 | EP 3745848 A1 | 09-12-2020 |
| | | US 2021087161 A1 | 25-03-2021 |
| | | WO 2019145557 A1 | 01-08-2019 |
| WO 2015132267 A1 | 11-09-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **S. ARCTANDER**. Perfume and Flavor Chemicals. Selbstverlag, 1969, vol. I,II
- **H. SURBURG** ; **J. PANTEN**. Common Fragrance and Flavor Materials. Wiley-VCH, 2006